# EUROPÄISCHE PATENTSCHRIFT

(11) **EP 0 646 588 B1**
(45) Veröffentlichungstag und Bekanntmachung des Hinweises auf die Patenterteilung: **26.01.2000**
(21) Anmeldenummer: 94115104.5
(22) Anmeldetag: 26.09.1994
(51) Int. Cl.: C07F 9/6568, C07C 45/50, C07F 15/00, B01J 31/28

(54) **Cyclische Verbindungen des dreiwertigen Phosphors**
Cyclic derivatives of trivalent phosphorus
Dérivés cycliques du phosphore trivalent

(30) Priorität: 30.09.1993 DE 4333307
(43) Veröffentlichungstag der Anmeldung: 05.04.1995
(73) Patentinhaber: Celanese Chemicals Europe GmbH, 60439 Frankfurt am Main (DE)
(72) Erfinder: Bahrmann, Helmut, Dr., D-46499 Hamminkeln (DE); Lappe, Peter, Dr., D-46539 Dinslaken (DE); Herrmann, Wolfgang A., Prof. Dr., D-85354 Freising (DE); Albanese, Guido, D-80993 München (DE); Manetsberger, Rainer Dr., D-82407 Wielenbach (DE)

(56) Entgegenhaltungen:
- EP-A- 0 627 439
- WO-A-87/07600
- WO-A-92/19630
- WO-A-93/15089
- US-A- 5 171 892

## Beschreibung

Die Erfindung betrifft neue cyclische Phosphor(III)-verbindungen, die als zweizähnige Liganden mit Metallen, insbesondere Metallen der 8. Gruppe des Periodensystems der Elemente, Komplexverbindungen bilden, sowie ein Verfahren zu ihrer Herstellung. Die Komplexverbindungen werden, homogen gelöst, als Bestandteile von Katalysatoren, insbesondere Hydroformylierungskatalysatoren, eingesetzt.

Komplexverbindungen, die als Zentralatom ein Metall der 8. Gruppe des Periodensystems der Elemente und als Liganden Phosphor(III)-verbindungen, Phosphine oder Phosphite und daneben gegebenenfalls noch weitere, zur Komplexbildung befähigte Gruppen enthalten, haben in den letzten Jahren zunehmend als Katalysatoren Bedeutung gewonnen. So erfolgt die technisch in großem Umfang ausgeübte Reaktion von Olefinen mit Synthesegas zu Aldehyden (Hydroformylierung) in Gegenwart von Katalysatorsystemen, die aus Kobalt und insbesondere aus Rhodium und Triphenylphosphin bestehen. Aber auch die Verwendung von Diphosphinen als Katalysatorbestandteil ist bekannt.

So offenbart WO-A-87 07600 einen Hydroformylierungsprozeß in Gegenwart von Diphosphinen als Liganden, wobei das Phosphoratom über eine Alkylengruppe mit einem aromatischen Rest verbunden ist. Die weiteren, am Phosphoratom gebundenen Reste können aliphatischer oder aromatischer Natur sein. Auch zur Hydrierung ungesättigter, Kohlenstoff-Kohlenstoff- oder Kohlenstoff-Sauerstoff-Mehrfachbindungen mit molekularem Wasserstoff haben sich Katalysatoren auf Basis Phosphine enthaltender Komplexverbindungen bewährt.

Aus WO93/15089 ist bekannt, daß Diphosphine, die neben einem ungesättigten phosphorhaltigen Rest auch eine gesättigte Dicyclohexylphosphinogruppe enthalten, als Bestandteil von Hydrierkatalysatoren eingesetzt werden können. Solche Katalysatoren lassen sich für die asymmetrische Hydrierung oder für die enantioselektive Wasserstoffverschiebung in prochiralen, alkylischen Systemen verwenden.

Auch US-A-5,171,892 offenbart Diphosphine, die als Bestandteil von Hydrierkatalysatoren verwendet werden können. Die in dieser Patentschrift offenbarten Diphosphine enthalten gesättigte Phospholanringe.

Ferner lehrt WO92/19630 die Verwendung von chiralen dreizähnigen Liganden als Katalysatorbestandteil für in Hydrierreaktionen eingesetzte Katalysatoren. Auch diese Liganden enthalten den gesättigten Phospholanring.

Üblicherweise liegen in den genannten Fällen die Liganden im Überschuß vor, so daß das Katalysatorsystem aus Komplexverbindung und freiem Ligand besteht. Entsprechend der Löslichkeit der Katalysatoren in organischen Medien erfolgen die Reaktionen in homogener Lösung.

Die bekannten katalytischen Verfahren haben sich in technischem Maßstab sehr gut bewährt. Dennoch bemüht man sich um eine weitere Vervollkommnung der bekannten Prozesse.

So versucht man, die Aktivität der Katalysatoren durch Modifizieren der Komplexliganden zu erhöhen und ihre Wirksamkeit zu verlängern, um den spezifischen Katalysatorbedarf zu vermindern. Darüber hinaus strebt man an, angepaßte Ligandensysteme zu entwickeln, die individuelle Probleme lösen. Als Beispiel sei die Steuerung der Regio- und Stereoselektivität chemischer Synthesen genannt, mit dem Ziel, reine Enantiomere aus prochiralen Ausgangsstoffen zu erhalten. Derartige Reaktionen gewinnen insbesondere auf dem Gebiet der Feinchemikalien und der Pharmazeutika immer größere Bedeutung.

Die Beeinflussung der Stereoselektivität ist mit einzähnigen Liganden nicht mehr oder nur unzureichend möglich. Daher werden zur Gewinnung reiner enantiomerer Produkte vorwiegend zweizähnige Liganden eingesetzt. Unter ihnen haben bisher vorwiegend Diphosphine Bedeutung erlangt. So werden z.B. zur selektiven Hydroformylierung α,β-ungesättigter Ester, wie Acrylsäureester als prochirale Verbindungen, Katalysatorsysteme auf Basis Kobalt/1,2-bis(diphenylphosphino)-ethan und Rhodium/1,4-bis(diphenylphosphino)-butan eingesetzt. Die Reaktionen erfordern energische Reaktionsbedingungen, d.h. Temperaturen von 120 bis 150°C und Drücke von 5 bis 10 MPa. Überdies haben sich die Katalysatorsysteme als wenig stabil erwiesen. Unter milderen Bedingungen werden mit dem Rhodiumkatalysator die Ausgangsverbindungen vorwiegend hydriert.

Es bestand daher die Aufgabe, als Bestandteile homogen löslicher Katalysatorsysteme geeignete Phosphorverbindungen zu entwickeln, in deren Molekül zwei zur Komplexbildung insbesondere mit Rhodium befähigte Phosphor(III)-atome enthalten sind. Die Katalysatorsysteme sollen hohe Aktivität und Regioselektivität besitzen und z.B. auch stereospezifische Synthesen ermöglichen.

Die Erfindung besteht dementsprechend in neuen Phosphorverbindungen der allgemeinen Formel

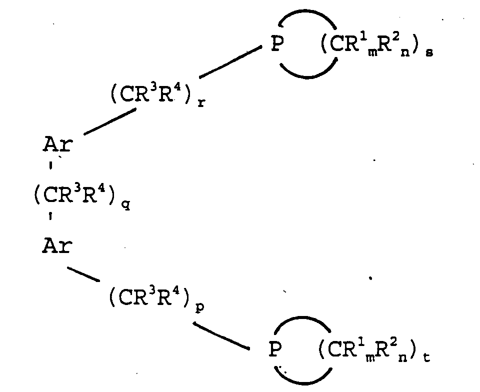

In der Formel sind die cyclischen Reste 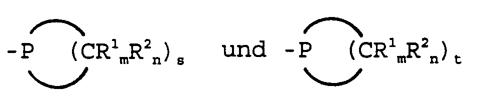 einfach oder mehrfach ungesättigt und fünf- oder sechsgliedrig, R³ und R⁴ sind gleich oder verschieden und bedeuten Wasserstoff oder den Methylrest, R¹ und R² sind ebenfalls gleich oder verschieden und stehen für Wasserstoff, einen Alkyl-, Aralkyl- oder Alkoxyrest mit jeweils 1 bis 5 Kohlenstoffatomen oder für ein Halogenatom, R¹ und R² können auch für annelierte Benzol- oder Naphthalinringe stehen. m und n sind gleich oder verschieden und stehen für die Zahlen 0 oder 1, s und t sind gleich oder verschieden und stehen für die Zahlen 4 oder 5. p, q, r sind gleich oder verschieden und stehen für die Zahlen 0, 1, 2 oder 3, Ar schließlich bedeutet einen substituierten oder nicht substituierten aromatischen Rest mit 6 bis 18 Kohlenstoffatomen. Ausgenommen ist die Verbindung, in der q gleich 0, Ar gleich 1,2-Phenylen, R³ und R⁴ gleich Wasserstoff, p und r gleich 1, s und t gleich 4, m gleich 1, n gleich 0 und R¹ einen annelierten Benzolring bedeutet und ebenso ist die Verbindung ausgenommen, in der q gleich 0, Ar gleich 5-tert.-Butyl-1,2-Phenylen, R³ und R⁴ gleich Wasserstoff, p und r gleich 1, s und t gleich 4, m gleich 1, n gleich 0 und R¹ einen annelierten Benzolring bedeutet.

Als Liganden werden solche Verbindungen, die der oben wiedergegebenen allgemeinen Formel entsprechen bevorzugt, in denen p und r jeweils für die Zahl 1 stehen und q 0 oder 1 ist und in denen R³ und R⁴ Wasserstoff bedeuten.

Der in der obigen Formel durch Ar charakterisierte aromatische Rest kann unsubstituiert oder substituiert sowie ein mehrkerniges oder kondensiertes Ringsystem sein. Bevorzugt steht Ar für einen jeweils nicht substituierten oder substituierten Phenylen-, Naphthylen-, Biphenylen- oder Binaphthylenrest.

Die in den neuen Verbindungen enthaltenen P(III)-atome sind Bestandteil gesättigter oder ungesättigter fünf- oder sechsgliedriger heterocyclischer Verbindungen. Bevorzugt sind Heterocyclen, die neben dem Phosphoratom noch vier Kohlenstoffatome enthalten, also Verbindungen, die sich von substituierten oder nicht substituierten Phospholanen, Phospholinen und insbesondere Phospholen ableiten. Dementsprechend stehen in der allgemeinen Formel s und t bevorzugt für 4. R¹ und R² sind gleich oder verschieden und bedeuten vorzugsweise jeweils einen geradkettigen oder verzweigten Alkylrest mit bis zu 5 Kohlenstoffatomen, den Benzyl-, Phenethyl-, Tolyl-, Xylolyl oder Mesitylrest. R¹ und R² können auch für annelierte Benzol- oder Naphthalinringe stehen. Die Reste entsprechender Phosphor enthaltender Heterocyclen leiten sich z.B. von 2,3-Benzophospholen, 2,3,4,5-Dibenzophospholen, 2,3-Indenophosphol ab.

Die neuen Verbindungen sind auf einfachem Wege in guten Ausbeuten zu erhalten. Eine bewährte Synthese ist die Phosphorylierung von Dihalogenalkylverbindungen der allgemeinen Formel 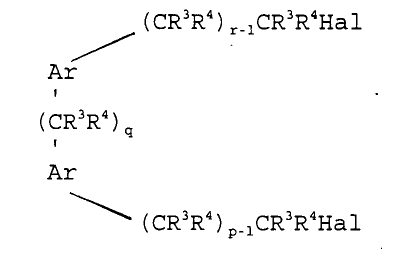 in der p, q, r, R³, R⁴ und Ar die vorstehend beschriebenen Bedeutungen haben und Hal ein Halogenatom, vorzugsweise Chlor oder Brom und insbesondere Brom bedeutet. Man gewinnt die Bromverbindungen z.B. aus den nichthalogenierten Grundsubstanzen durch Bromierung mit Hilfe von N-Bromsuccinimid.

Als Phosphorylierungsreagenz setzt man mit Erfolg Alkalimetallverbindungen mit heterocyclischem Phosphoranion ein. Ihre Herstellung erfolgt durch Umsetzung der am Phosphoratom phenylierten Heterocyclen mit einem Alkalimetall, vorzugsweise Natrium oder Kalium und insbesondere mit einer Natrium-/Kaliumlegierung. Das Metallderivat ergibt, mit der Dihalogenverbindung umgesetzt, die gewünschten cyclischen Phosphorverbindungen.

Die erfindungsgemäßen Phosphorverbindungen bilden als zweizähniger Ligand Komplexverbindungen mit zahlreichen Metallatomen. Unter ihnen haben die Komplexverbindungen des Rhodiums besondere Bedeutung als hochaktive Katalysatoren für die Niederdruckhydroformylierung von Olefinen zu Aldehyden. Die Umsetzung ergibt einen sehr hohen Anteil geradkettiger Verbindungen gegenüber lediglich untergeordneten Mengen verzweigtkettiger Isomerer. Als Katalysatorkomponenten besonders bewährt haben sich die Verbindungen 2,2'-Bis(3,4-dimethylphospholmethylen)-1,1'-biphenyl und 2,2'-Bis(3,4-dimethylphospholmethylen)-1,1'-binaphtyl.

Die Hydroformylierung von Olefinen unter Verwendung von Katalysatorsystemen aus Rhodium und den neuen Phosphor(III)-verbindungen erfolgt bei Drücken von etwa 0,1 bis etwa 6 MPa, vorzugsweise 0,5 bis 3 MPa und insbesondere 1,5 bis 2,0 MPa. Die Reaktionstemperatur kann etwa 20 bis etwa 200°C betragen, bevorzugt wird der Bereich von etwa 50 bis etwa 170°C und insbesondere etwa 80 bis etwa 150°C.

Der Katalysator wird dem Reaktionsgemisch präformiert, zweckmäßig in einem Lösungsmittel gelöst, zugeführt oder aber aus den Komponenten unter den Bedingungen der Hydroformylierungsreaktion im Gemisch der Reaktanten gebildet. Sowohl bei der vorherigen, als auch im Falle der in-situ-Herstellung des Katalysators kann das Rhodium als Metall in fein verteilter Form oder als Verbindung, z.B. den Oxiden, den Salzen anorganischer oder organischer Säuren oder als Carbonyl eingesetzt werden.

Die erforderlichen Rhodiummengen sind sehr gering, schon etwa 1 x 10⁻⁶ mol Rh je mol Olefin sind wirksam. Aus wirtschaftlichen Gründen, Steigerung der Reaktionsgeschwindigkeit, wendet man jedoch bevorzugt etwa 1 x 10⁻² bis etwa 1 x 10⁻⁵ mol und insbesondere 1 x 10⁻² bis 1 x 10⁻⁴ mol Rh je mol Olefin an. Das molare Verhältnis von Rhodium zu Ligand im Reaktionsgemisch beträgt etwa 1 zu 1 bis 200, vorzugsweise etwa 1 zu 2 bis 10 und insbesondere etwa 1 zu 2,5 bis 4.

Synthesegas und Olefin werden im Maße ihres Verbrauchs dem Reaktor kontinuierlich zugeführt. Das molare Verhältnis von Kohlenmonoxid und Wasserstoff im Synthesegas kann in weiten Bereichen variieren und etwa 1 zu 10 bis etwa 10 zu 1 betragen, wobei die Art des umzusetzenden Olefins Einfluß auf das zu wählende Verhältnis haben kann. Bevorzugt werden Gemische, die Kohlenmonoxid und Wasserstoff im Verhältnis von etwa 1 zu 2 bis etwa 2 zu 1 und insbesondere etwa 1 zu 1,5 bis 1,5 zu 1 enthalten. Synthesegas wird, bezogen auf Olefin, immer im Überschuß eingesetzt, ein molarer Überschuß von Synthesegas (CO+H₂), bezogen auf Olefin von etwa 0,5 bis etwa 20, ist gebräuchlich, von etwa 1 bis etwa 10 bevorzugt.

Die Umsetzung kann in Gegenwart von Lösungsmitteln durchgeführt werden, die unter den Reaktionsbedingungen inert gegenüber Reaktanten und Produkten sind. Zu diesen Lösungsmitteln zählen z.B. aromatische Kohlenwasserstoffe wie Benzol, Toluol, Xylol, aliphatische Kohlenwasserstoffe wie Pentane, Kerosin und Mineralöle sowie Alkohole, Ether und Ester.

Die Hydroformylierung erfolgt in konventionellen Reaktoren, sie kann kontinuierlich oder absatzweise erfolgen. Bei kontinuierlicher Reaktionsführung wird das Produkt laufend zusammen mit nicht umgesetztem Synthesegas abgezogen, aus der Gasphase durch Kondensation abgeschieden und konventionell weiterbehandelt, z.B. destillativ gereinigt. Das Synthesegas wird in die Reaktionszone zurückgeführt. Um die Aktivität des Katalysatorsystems auf gleichmäßig hohem Niveau zu halten kann es sich empfehlen, von Zeit zu Zeit Katalysator aus dem Reaktor abzuziehen und durch frischen Katalysator zu ersetzen.

Die neue Klasse zweizähniger Liganden wird, zusammen mit Rhodium, mit Erfolg bei der Hydroformylierung unterschiedlicher Olefine eingesetzt. Zu ihnen gehören α-Olefine mit 2 bis 20 Kohlenstoffatomen, die unter Reaktionsbedingungen inerte Substituenten enthalten können. Beispiele für geeignete Olefine sind Ethylen, Propylen, Buten-1, Buten-2, ferner die Pentene, Hexene und Heptene. Besondere Bedeutung haben die neuen Liganden wegen ihrer Stereospezifität bei der Hydroformylierung prochiraler Olefine.

Die folgenden Beispiele erläutern die Erfindung, ohne sie auf die näher beschriebenen Ausführungsformen zu beschränken.

### Beispiele

### 1.) Herstellung von 2,2'-Bis(3,4-Dimethylphospholmethylen)-1,1'-biphenyl

### (a) 3,4-Dimethyl-1-Phenylphosphol (nach A. Breque et al., Synth., 1981, 12, 984)

In einem 1000 ml Rührkolben werden unter Stickstoff 44,7 g (250 mmol) Phenyldichlorphosphin und 66,9 g (250 mmol) Phenyldibromphosphin 30 min bei Raumtemperatur zusammen gerührt. Dann gibt man zu der homogenen Lösung unter Eiskühlung 42,0 g (511 mmol) Dimethylbutadien und rührt unter weiterer Kühlung 24 h. Die entstehende Suspension läßt man weitere 10 d bei Raumtemperatur stehen. Sie wandelt sich dabei vollständig in eine weiße, kristalline Masse um, die im Kolben in möglichst kleine Stücke zerteilt und mit 300 ml Hexan und 200 ml Dichlormethan überschichtet wird.

Durch Einblasen von Stickstoff über einen Zeitraum von 30 min wird die Suspension von überschüssigem Dimethylbutadien befreit. Unter starkem Rühren fügt man bei Raumtemperatur tropfenweise eine Lösung von 103 g (1094 mmol) 2-Methylpyridin in 100 ml Dichlormethan zu. Im Verlauf von 24 h entsteht eine Mischung, die sich in zwei flüssige Phasen trennen läßt. Nach der Hydrolyse mit 100 ml 3 n Salzsäure trennt man die organische Phase ab und wäscht dreimal mit 100 ml Wasser bis zur Neutralität. Man trocknet über Natriumsulfat, filtriert und dampft ein. Die zurückbleibende Flüssigkeit wird mit 300 ml Hexan extrahiert, der Extrakt filtriert und eingedampft. Das so erhaltene gelbe Öl bedarf keiner weiteren Reinigung.
Ausbeute: 71,17 g (378 mmol, 74,15 % der Theorie).
³¹P-NMR (161,8 MHz, DC₂Cl₂): = -1,3 ppm (s, 1P).

### (b) 2,2'-Dimethyl-1,1'-biphenyl (nach C.W. Kohlpaintner, Diplomarbeit 1990, Technische Universität München)

In einem 500 cm³-Kolben werden zu 18.59 g (0.75 mol) Mg-Späne in 200 ml Tetrahydrofuran über einen Zeitraum von 1 h unter Rühren 70 ml (0.6 mol) 2-Chlortoluol und 2 ml 1,2-Dibromethan getropft, wobei Graufärbung und schwache Erwärmung erfolgen. Anschließend erhitzt man 18 h unter Rückfluß. Die Suspension wird nach dem Abkühlen über Glaswolle filtriert und zu einer Lösung von 70 ml (0.6 mol) 2-Chlortoluol und 3.0 g (4.5 mmol) cis-Dichloro-bis(triphenylphosphin)nickel(II) in 250 ml Tetrahydrofuran über 2 h bei Raumtemperatur getropft. Nach 20 h Erhitzen unter Rückfluß wird die Flüssigkeit in einen 1000-cm³-Scheidetrichter gegeben und durch vorsichtige Zugabe von 400 ml Toluol und 200 ml einer gesättigten, wäßrigen Ammoniumchloridlösung langsam abgekühlt. Die organische Phase wird abgetrennt und sukzessive mit 250 ml 10 Gew.-%iger HCl und dreimal mit je 125 ml Wasser gewaschen. Das Lösungsmittel wird im Vakuum einer Ölpumpe entfernt, wobei ein braun-gelbes Öl zurückbleibt. Destillation bei 66,7 bis 133,3 Pa liefert das erwünschte Produkt als farblose Flüssigkeit in einem Siedetemperaturbereich von 75 - 77°C.

Ausbeute: 77.31 g (70.7 % der Theorie).

GC-Massenspektrum (AS 60, Retentionszeit 12.796 min): m/z = 182 (55%, [(M)⁺.]), 167 (100%, [(M-CH₃)⁺.]), 152 (20%, [(C₁₂H₈)⁺.]), 128 (10%), 115 (12%), 89 (18%), 76 (10%, [(C₆H₄)⁺.]), 63 (15%), 39 (18%), 27 (12%).

### (c) 2,2'-Bis(bromomethylen)-1,1'-biphenyl (Nach W. Wenner, J. Org. Chem. 1952, 17, 523-528)

Zu 7.1 g (0.039 mol) 2,2'-Bismethyl-1,1'-biphenyl in 15 ml Tetrachlorkohlenstoff werden 20.0 g (0.11 mol) N-Bromosuccinimid und 0.2 g Azo-iso-butyronitril gegeben und unter leichtem Rückfluß erhitzt. Nach 9 h wird das Succinimid bei Raumtemperatur über eine G3 Glasfritte abfiltriert und die zurückbleibende Flüssigkeit bei 40°C im Ölpumpenvakuum zu einem zähen, gelben Öl eingeengt. Das Produkt wird durch Umkristallisieren aus 15 ml Ligroin in Form feiner, weißer Kristalle erhalten, die über eine G4 Glasfritte abfiltriert und im Ölpumpenvakuum getrocknet werden.
Ausbeute: 11.5 g (84 % der Theorie)
Schmelzpunkt: 82°C

GC-Massenspektrum (SA60, Retentionszeit 17.273 min): m/z = 340 (6%, [(M)⁺.]), 259 (15%, [(M-Br)⁺.]), 179 (100%, [(M-2*Br)⁺.]), 152 (12%, [(C₁₂H₈)⁺.].

| Elementaranalyse: (C₁₄H₁₂Br₂; 340.07) | | | |
|---|---|---|---|
| Ber. | C 49.45 | H 3.55 | Br 47.00, |
| Gef. | C 49.20 | H 3.58 | Br 46.09 |

### (d) (3.4-Dimethylphospholyl)natrium/kalium

1.88 g (10 mmol) 1-Phenyl-3,4-dimethylphosphol in 40 ml Tetrahydrofuran werden langsam mit 0.62 g (20 mmol) Natrium/Kalium-Legierung bei Raumtemperatur portionsweise unter starkem Rühren versetzt. Nach 2 h wird überschüssige Metallegierung über Glaswolle filtriert. Die dunkelbraune Suspension wird auf -30°C gekühlt und mit 0.16 g (0.19 ml, 17 mmol) 2-Chloro-2-methylpropan versetzt. Erwärmen innerhalb 1 h führt zu einer dunkelroten Suspension, die ohne Isolierung des Produkts weiterverarbeitet wird. Nach ³¹P-NMR erfolgt die Umsetzung quantitativ.
³¹P-{1H}-NMR (CDCl₃): δ = 55.40 ppm (s, 1P), 52.36 ppm (s, 1P).

### (e) 2,2'-Bis(3,4-Dimethylphospholmethylen)-1,1'-biphenyl

Zu 15.8 g (84 mmol) in 150 ml Tetrahydrofuran suspendiertem (3,4-Dimethylphospholyl)-natrium/kalium werden 12.0 g (35 mmol) 2,2'-Bis(bromomethyl)-1,1'-biphenyl in 30 ml Tetrahydrofuran getropft. Nach 12 h Rühren verfärbt sich die Suspension von dunkelrot nach hellbraun und das Lösungsmittel wird im Vakuum einer Ölpumpe entfernt. Der zurückbleibende braune Rückstand wird in Toluol aufgenommen, wobei KBr und NaBr ausfallen. Die überstehende, klare Lösung wird zu einem dunkelbraunen Öl eingeengt und das Produkt durch Umkristallisation mit 10 ml Ethanol als weißes, mikrokristallines Pulver gewonnen.
Ausbeute: 8.52 g (21 mmol, 60 % der Theorie)
Schmelzpunkt: 120°C
Massenspektrum (CI): m/e = 402.2 (36.99%, [(M)⁺.]), 291.2 (14.87%, [(M-C₄H₁₀P)⁺.]), 179.1 (4.14%, [(M-2*C₄H₁₀P)⁺.]), 154 (2.66%, [(M-2*C₅H₁₁P⁺.])
³¹P-{1H}-NMR (CDCl₃): δ = 3.99 (s, 2P)
¹H-NMR (CDCl₃): δ = 0.89 (t, 6H, -CH₃, 2J(H,H) = 7.4 Hz), 1.19 (t, 6H, -C'H₃, ²J(H,H) = 7.4 Hz), 1.54 (dd, 2 H, - CHₐH_{b}-, ²J(H,H) = 14.8 Hz, ²J(P,H) = 6.78 Hz), 1.97 (d, 2 H, -CHₐH_{b}-, ²J(H,H) = 20.34 Hz) 3.55 (7, 2 H, :CH-, ²J(P,H) = 6.78), 3.67 (t, 2 H, :C'H-, ²J(P,H) = 6.78), 8.6 (m, 8 H, Aromat)

| Elementaranalyse: (C₂₆H₂₈P₂; 402.26) | | | |
|---|---|---|---|
| Ber. | C 77.60 | H 7.00 | P 15.39 |
| Gef. | C 77.08 | H 7.05 | P 15.68 |

### 2.) Herstellung von 2,2'-Bis(3,4-Dimethylphospholmethylen)-1,1'-binaphthyl

### (a) 2,2'-Bismethyl-1,1'-binaphthyl

83.8 g (0.379 mol) 1-Bromo-2-methylnapthalin, gelöst in 150 ml Ether und 150 ml Benzol, werden bei Raumtemperatur langsam zu 10 g (0,415 mol) Magnesiumspänen (zuvor mit wenigen Tropfen 1,2-Dibromethan versetzt), suspendiert in 30 ml Ether, getropft. Das Zutropfen des halogenierten Aromaten erfolgt über einen Zeitraum von 4 h. Die Zutropfgeschwindigkeit wird dabei so gewählt, daß der Reaktionsansatz immer handwarm bleibt. Anschließend wird das Reaktionsgemisch noch eine Stunde am Rückfluß erhitzt und weiter über Nacht bei Raumtemperatur gerührt. Die Suspension wird aus einem Tropftrichter portionssweise zu 74 g (0,335 mol) 1-Bromo-2-methylnaphthalin, gelöst in 250 ml Ether und 2,5 g (3,8 mmol) [(PPh₃)₂Ni]Cl₂ (Kupplungskatalysator) gegeben. Darauf erhitzt man 24 h unter Rückfluß, kühlt auf Raumtemperatur ab und hydrolysiert mit 200 ml Wasser und 200 ml 20 Gew.-%iger Salzsäure. Die von der wäßrigen Phase getrennte organische Phase wird noch zweimal mit 200 ml Wasser nachgewaschen, erneut abgetrennt und mit Magnesiumsulfat getrocknet. Die organischen Lösungsmittel werden im Vakuum abgezogen, der Rückstand wird über eine kleine Vigreux-Kolonne bei Drücken < 1,3 Pa destilliert. Die gewünschte Verbindung geht unter den gegebenen Bedingungen in einem Bereich von 169 - 173°C als grünes Öl über, das mit 10 ml Aceton bei -18°C umkristallisiert wird.
Ausbeute: 74,9 g (0,265 mol, 69 % der Theorie)

### (b) 2,2'-Bisbromomethyl-1,1'-binaphthyl (nach W. Wenner, J. Org. Chem. 1952, 17, 523-528)

28,2 g (0,1 mol) 2,2'-Dimethyl-1,1'-binaphthyl werden mit 35,6 g (0,2 mol) N-Bromosuccinimid in 65 ml Tetrachlorkohlenstoff suspendiert. Unter Rückfluß werden 0,43 g Azo-iso-butyronitril in mehreren Anteilen über 4 h zugegeben und weitere 9 h unter Rückfluß erhitzt. Nach Abfiltrieren des Succinimids und Einengen des Filtrats bleibt ein gelbes, zähes Öl zurück. Es wird aus 20 ml Ligroin (Leichtbenzinmischung) umkristallisiert und der erwünschte weiße Feststoff abfiltriert.
Ausbeute: 35,55 g (0,08 mol, 80 % der Theorie)
¹H-NMR (CDCl₃): δ = 8,0 - 9,0 (m, aromat., 12 H), 5,3 ppm (s, CH₂, 4H)

### (c) 2,2'-Bis(3,4-Dimethylphospholmethylen)-1,1'-binaphthyl

Zu einer Suspension von 5,0 g (0,045 mol) 3,4-Dimethylphospholylanion in 120 ml Tetrahydrofuran, werden 9,9 g (0,022 mol) 2,2'-Bisbromomethyl-1,1'-binaphthyl in 20 ml Tetrahydrofuran über 2 h zugetropft. Nach 18 h Rühren bei Raumtemperatur wird das Lösungsmittel vollständig entfernt und der weiß-braune Rückstand mit 80 ml Toluol in einer Soxhlet-Apparatur extrahiert. Anschließendes Einengen führt zu einem braunen Öl, aus dem sich das Produkt als hellbrauner Feststoff durch Umkristallisieren mit 30 ml n-Hexan gewinnen läßt.
Ausbeute: 5,46 g (0,01 mol, 49 % der Theorie)
³¹P-{1H}-NMR (CDCl₃): δ = -5,4 ppm (s, 2P)
Massenspektrum (CI): m/e = 503,1 (5,6%, [(M)⁺.]), 391,1 (15,1% [(M-C₄H₁₀P)⁺.]), 281,1, (15,4%, [(M-2*C₄H₁₀P)⁺.]), 154 (2.66%, [(M-2*C₅H₁₁P)⁺.]).

## Patentansprüche

1. Verbindungen der allgemeinen Formel 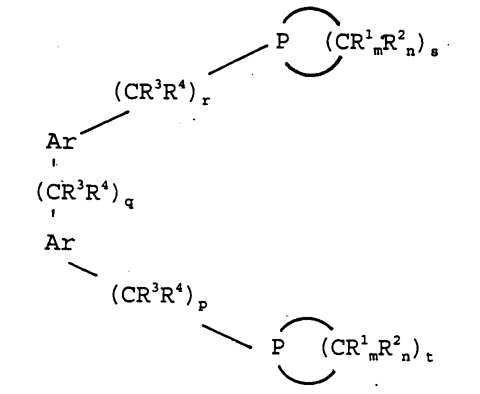 in der die Reste 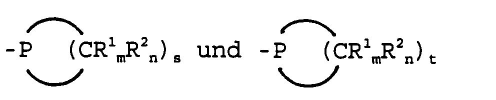 einfach oder mehrfach ungesättigt und fünf- oder sechsgliedrig sind und R³ und R⁴ gleich oder verschieden sind und Wasserstoff oder den Methylrest bedeuten, R¹, R² gleich oder verschieden sind und für Wasserstoff, einen Alkyl-, Aralkyl- oder Alkoxyrest mit jeweils 1 bis 5 Kohlenstoffatomen oder für ein Halogenatom stehen oder einen annelierten Benzol- oder Naphthalinring bedeuten, m, n gleich oder verschieden sind und für die Zahlen 0 oder 1 stehen, s, t gleich oder verschieden sind und für die Zahlen 4 oder 5 stehen, p, q, r gleich oder verschieden sind und für die Zahlen 0, 1, 2 oder 3 stehen und Ar einen substituierten oder nicht substituierten aromatischen Rest mit 6 bis 18 Kohlenstoffatomen bedeutet, mit Ausnahme der Verbindung, in der q gleich 0, Ar gleich 1,2-Phenylen, R³ und R⁴ gleich Wasserstoff, p und r gleich 1, s und t gleich 4, m gleich 1, n gleich 0 und R¹ einen annelierten Benzolring bedeutet und mit Ausnahme der Verbindung, in der q gleich 0, Ar gleich 5-tert.-Butyl-1,2-Phenylen, R³ und R⁴ gleich Wasserstoff, p und r gleich 1, s und t gleich 4, m gleich 1, n gleich 0 und R¹ einen annelierten Benzolring bedeutet.

2. Verbindungen nach Anspruch 1, dadurch gekennzeichnet, daß die Reste R³ und R⁴ für Wasserstoff stehen.

3. Verbindungen nach Anspruch 1 oder 2, dadurch gekennzeichnet, daß die Reste R¹ und R² für Wasserstoff oder den Methylrest stehen.

4. Verbindungen nach einem oder mehreren der Ansprüche 1 bis 3, dadurch gekennzeichnet, daß Ar ein Phenylen-, ein Naphthylen-, ein 1,1'-Biphenylen- oder ein 1,1'-Binaphthylenrest ist.

5. Verbindungen nach einem oder mehreren der Ansprüche 1 bis 4, dadurch gekennzeichnet, daß s und t für die Zahl 4 steht.

6. Verbindungen nach einem oder mehreren der Ansprüche 1 bis 5, dadurch gekennzeichnet, daß p und r für die Zahl 1 steht.

7. Verbindungen nach einem oder mehreren der Ansprüche 1 bis 6, dadurch gekennzeichnet, daß q für die Zahlen 0 oder 1 steht.

8. Die chemische Verbindung 2,2'-Bis(3,4-dimethylphospholmethylen)-1,1'-biphenyl.

9. Die chemische Verbindung 2,2'-Bis(3,4-dimethylphospholmethylen)-1,1'-binapthyl.

10. Verfahren zur Herstellung von Verbindungen nach einem oder mehreren der Ansprüche 1 bis 9, dadurch gekennzeichnet, daß man Verbindungen der allgemeinen Formel 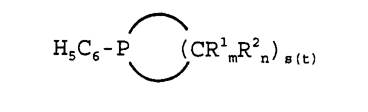 in der R¹, R², m, n, s und t die vorstehend wiedergegebenen Bedeutungen haben, mit Alkalimetallen, mit Ausnahme von Lithium, umsetzt und darauf mit Verbindungen der allgemeinen Formel 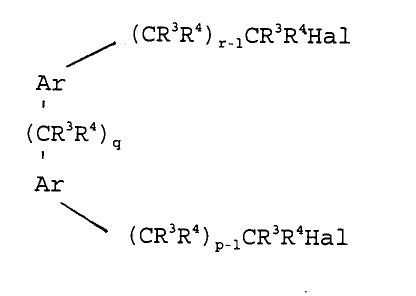 in der X, Ar, R³, R⁴, p, q und r die vorstehend wiedergegebene Bedeutung haben und Hal Halogen, insbesondere Cl oder Br ist, reagieren läßt.

11. Verfahren nach Anspruch 10, dadurch gekennzeichnet, daß als Alkalimetalle eine Natrium-Kaliumlegierung eingesetzt wird.

12. Verwendung von Verbindungen nach einem oder mehreren der Ansprüche 1 bis 9 als Bestandteil homogen löslicher Katalysatorsysteme.

## Claims

1. Compounds of the formula 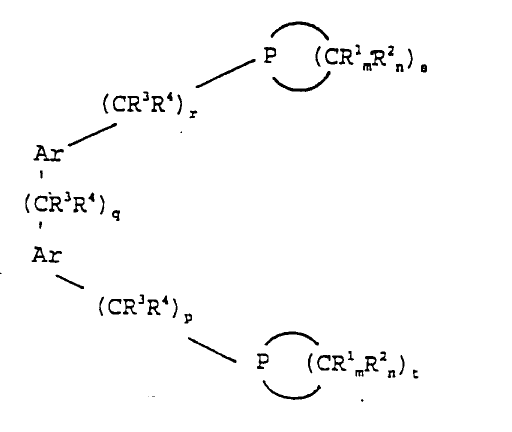 in which the radicals 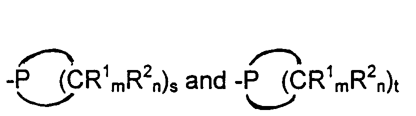 are monounsaturated or polyunsaturated and five or six-membered and R³ and R⁴ are identical or different and stand for hydrogen or the methyl radical, R¹, R² are identical or different and stand for hydrogen, an alkyl, aralkyl or alkoxy radical having in each case 1 to 5 carbon atoms or for a halogen atom or represent a fused benzene or naphthalene ring, m, n are identical or different and stand for the integers 0 or 1, s, t are identical or different and stand for the integers 4 or 5, p, q, r are identical or different and stand for the integers 0, 1, 2 or 3 and Ar is a substituted or non-substituted aromatic radical having 6 to 18 carbon atoms, with the exception of the compound in which q equals 0, Ar is 1,2-phenylene, R³ and R⁴ are hydrogen, p and r equal 1, s and t equal 4, m equals 1, n equals 0 and R¹ stands for a fused benzene ring and with the exception of the compound in which q equals 0, Ar equals 5-tert.-butyl-1,2-phenylene, R³ and R⁴ stand for hydrogen, p and r equal 1, s and t equal 4, m equals 1, n equals 0 and R¹ is a fused benzene ring.

2. Compounds according to claim 1, characterised in that the radicals R³ and R⁴ stand for hydrogen.

3. Compounds according to claim 1 or 2, characterised in that the radicals R¹ and R² stand for hydrogen or the methyl radical.

4. Compounds according to one or more of claims 1 to 3, characterised in that Ar is a phenylene, a naphthylene, a 1,1'-biphenylene or a 1,1'-binaphthylene radical.

5. Compounds according to one or more of claims 1 to 4, characterised in that s and t stand for the integer 4.

6. Compounds according to one or more of claims 1 to 5, characterised in that p and r stand for the integer 1.

7. Compounds according to one or more of claims 1 to 6, characterised in that q stands for the integer 0 or 1.

8. The chemical compound 2,2'-bis(3,4-dimethylphospholmethylene)-1,1'-biphenyl.

9. The chemical compound 2,2'-bis(3,4-dimethylphospholmethylene)-1,1'-binaphthyl.

10. A process for preparing compounds according to one or more of claims 1 to 9, characterised in that compounds of the formula 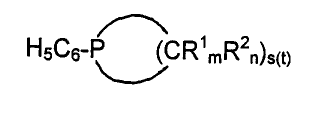 in which R¹, R², m, n, s and t have the aforementioned meanings, are reacted with alkali metals with the exception of lithium and are then allowed to react with compounds of the formula 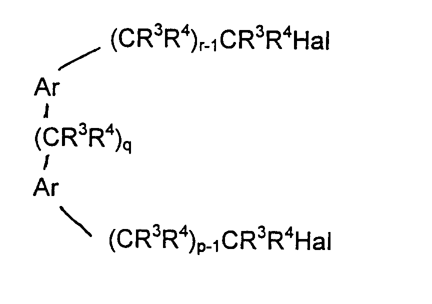 in which X, Ar, R³, R⁴, p, q and r have the aforementioned meanings and Hal is halogen, in particular Cl or Br.

11. The process according to claim 10, characterised in that a sodium-potassium alloy is used as alkali metals.

12. The use of compound according to one or more of claims 1 to 9 as part of homogeneously soluble catalyst systems.

## Revendications

1. Composés de la formule générale 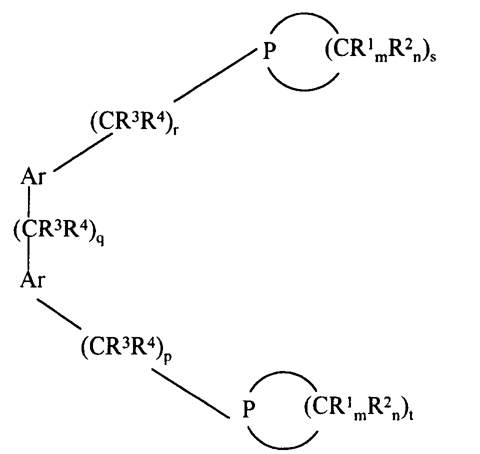 dans laquelle les restes 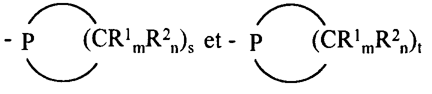 sont une ou plusieurs fois insaturés et à 5 ou 6 éléments et R³ et R⁴ sont identiques ou différents et représentent un atome d'hydrogène ou le reste méthyle, R¹, R² sont identiques ou différents et représentent un atome d'hydrogène, un reste alkyle, aralkyle ou alcoxy ayant à chaque fois de 1 à 5 atomes de carbone ou un atome d'halogène ou représentent un cycle benzène ou naphtalène condensé, m, n sont identiques ou différents et représentent les nombres 0 ou 1, s, t sont identiques ou différents et représentent les nombres 4 ou 5, p, q, r sont identiques ou différents et représentent les nombres 0, 1, 2 ou 3 et Ar représente un reste aromatique substitué ou non substitué ayant de 6 à 18 atomes de carbone, à l'exception du composé dans lequel q est égal à 0, Ar est le 1,2-phénylène, R³ et R⁴ sont tous deux un atome d'hydrogène, p et r sont égaux à 1, s et t sont égaux à 4, m est égal à 1, n est égal à 0 et R¹ représente un cycle benzène condensé et à l'exception du composé dans lequel q est égal à 0, Ar est le 5-tert.-butyl-1,2-phénylène, R³ et R⁴ sont tous deux un atome d'hydrogène, p et r sont égaux à 1, s et t sont égaux à 4, m est égal à 1, n est égal à 0 et R¹ représente un cycle benzène condensé.

2. Composés selon la revendication 1, caractérisés en ce que les restes R³ et R⁴ représentent un atome d'hydrogène.

3. Composés selon la revendication 1 ou 2, caractérisés en ce que les restes R¹ et R² représentent un atome d'hydrogène ou le reste méthyle.

4. Composés selon l'une ou plusieurs quelconques des revendications 1 à 3, caractérisés en ce que Ar est un reste phénylène, un reste naphtylène, un reste 1,1'-biphénylène ou un reste 1,1'-binaphtylène.

5. Composés selon l'une ou plusieurs quelconques des revendications 1 à 4, caractérisés en ce que s et t représentent le nombre 4.

6. Composés selon l'une ou plusieurs quelconques des revendications 1 à 5, caractérisés en ce que p et r représentent le nombre 1.

7. Composés selon l'une ou plusieurs quelconques des revendications 1 à 6, caractérisés en ce que q représente les nombres 0 ou 1.

8. Composé chimique 2,2'-bis(3,4-diméthylphospholméthylène)-1,1'-biphényle.

9. Composé chimique 2,2'-bis(3,4-diméthylphospholméthylène)-1,1'-binaphtyle.

10. Procédé pour la préparation de composés selon l'une ou plusieurs quelconques des revendications 1 à 9, caractérisé en ce que l'on fait réagir des composés de la formule générale 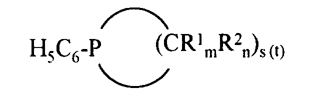 dans laquelle R¹, R², m, n, s et t ont la signification indiquée précédemment avec des métaux alcalins à l'exception du lithium et on laisse réagir après cela avec des composés de la formule générale 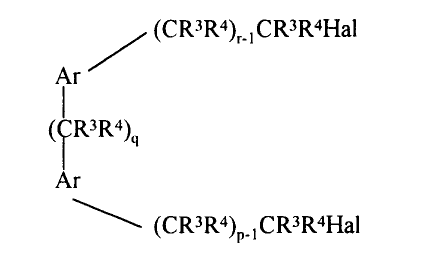 dans laquelle X, Ar, R³, R⁴, p, q et r ont la signification indiquée précédemment et Hal est un atome d'halogène, en particulier Cl ou Br.

11. Procédé selon la revendication 10, caractérisé en ce que l'on utilise comme métaux alcalins un alliage de sodium-potassium.

12. Utilisation de composés selon l'une ou plusieurs quelconques des revendications 1 à 9 comme constituants de systèmes de catalyseurs dissous de manière homogène.
